# EUROPÄISCHE PATENTSCHRIFT

(11) **EP 2 201 886 B1**
(45) Veröffentlichungstag und Bekanntmachung des Hinweises auf die Patenterteilung: **20.03.2013**
(21) Anmeldenummer: 09401039.4
(22) Anmeldetag: 16.11.2009
(51) Int. Cl.: A61B 19/00, A47L 15/00

(54) **Spülgutträger mit einem Anschlussstück für schlauchförmiges Reinigungsgut**
Wash rack with an attachment piece for tubular objects to be cleaned.
Porte-vaisselle doté d'une pièce de raccordement pour marchandise de nettoyage tubulaire

(30) Priorität: 18.12.2008 DE 102008062760
(43) Veröffentlichungstag der Anmeldung: 30.06.2010
(73) Patentinhaber: Miele & Cie. KG, 33332 Gütersloh (DE)
(72) Erfinder: Knorr, Jochen, 33659 Bielefeld (DE); Thorman, Franz, 33649 Bielefeld (DE)

(56) Entgegenhaltungen:
- EP-A1- 0 483 059
- EP-A2- 1 266 604
- EP-A2- 1 946 692
- DE-A1- 3 413 386
- DE-A1- 4 323 816

## Beschreibung

Die Erfindung betrifft einen Spülgutträger nach dem Oberbegriff von Anspruch 1.

Ein Spülgutträger dieser Art ist aus der DE 10 2007 003 894 A1 bekannt. Das Anschlussstück dient zur Ankopplung des schlauchförmigen Reinigungsguts an den Spülflüssigkeits- und Trocknungskreislauf einer Spülmaschine. Der dort beschriebene, als Korb ausgebildete Träger, ist mit einem Anschlussstück ausgestattet, welches lediglich in Richtung der Spülbehälterwand beweglich gelagert, aber ansonsten fest am Korb montiert ist. Will man das Reinigungsgut mit den am Anschlussstück vorhandenen Anschlussstutzen verbinden, muss man entweder den gesamten Korb aus der Maschine herausnehmen, oder die Verbindung bei einem im Spülbehälter der Maschine befindlichen Korb vornehmen. Dies erschwert die Handhabung. Ein weiterer Nachteil ergibt sich durch Toleranzen, welche sich korbseitig bei der Fertigung des Korbes, bei der Montage des Anschlussstücks und spülmaschinenseitig beim Aufbau der Korbführung sowie bei der Positionierung der Kupplungsvorrichtung einstellen. Die bekannte Anordnung des Anschlussstücks lässt nur einen geringen Ausgleich solcher Toleranzen zu, so dass es zu Schwierigkeiten bei der Verbindung von Anschlussstück und Kupplungsvorrichtung kommen kann.

Die DE4323816A1 beschreibt eine Vorrichtung zur Intensivreinigung von Innenkanäle aufweisenden ärztlichen Instrumenten, bei der eine mit den ärztlichen Instrumenten bestückbare Kassette in eine Reinigungskammer einführbar ist. Bei vollständig eingeschobener Kassette sind die ärztlichen Instrumente an der rückwärtigen Wand der Kassette über Anschlussflansche mit Zuleitungen verbunden, so dass deren Innenkanäle mit Wasser versorgt werden können. Zwischen den Instrumenten und den Anschlussflanschen sind verschiedene Arten von Adaptern zwischengeordnet, welche abhängig von der Instrumentenart ausgewählt und vor dem Einschieben der Kassette auf die Adapterflansche aufgesetzt werden können, um die Ankopplung von Instrumenten mit unterschiedlichen Gestaltungen zu ermöglichen. Der Erfindung stellt sich somit das Problem, bei einem Spülgutträger der eingangs genannten Art die Handhabung zu vereinfachen und außerdem eine sichere Verbindung des Anschlussstücks mit einer Kupplungsvorrichtung zu ermöglichen.Erfindungsgemäß wird dieses Problem durch einen Spülgutträger mit den Merkmalen des Patentanspruchs 1 gelöst.

Die mit der Erfindung erreichbaren Vorteile werden dadurch erreicht, dass das Anschlussstück lösbar an der Seitenwand befestigt ist. Hierdurch besteht einerseits die Möglichkeit, die notwendige Verbindung von Reinigungsgut und Anschlussstück losgelöst vom Spülgutträger vorzunehmen. Hierdurch wird die Handhabung erleichtert. Andererseits lässt diese Art der Befestigung ein gewisses Spiel zu, welches aufgrund der fertigungsbedingten Toleranzen notwendig ist, um eine sichere Verbindung des Anschlussstücks mit der Kupplungsvorrichtung zu ermöglichen.

Vorteilhafte und zweckmäßige Ausgestaltungen und Weiterbildungen der Erfindung ergeben sich aus den nachfolgenden Unteransprüchen.

So wird beispielsweise eine einfache Befestigung des Anschlussstücks ermöglicht, wenn dieses in der Betriebslage des Spülgutträgers in vertikaler Richtung in eine Aussparung der Seitenwand einsetzbar ist.

Eine einfache Fertigung wird dadurch ermöglicht, dass das Anschlussstück aus Blech hergestellt ist.

In einer besonders vorteilhaften Ausführungsform besitzt das Anschlussstück mindestens eine Haltelasche mit einer Öffnung, welche von einem in der Aussparung angeordneten, sich in der Betriebslage des Spülgutträgers in vertikaler Richtung erstreckenden Dorn aufnehmbar ist. Hierdurch ist eine Fixierung durch einfaches Einhängen möglich. Dabei wird eine sichere Halterung erreicht, wenn eine obere Haltelasche und eine untere Haltelasche vorgesehen sind, wobei mindestens eine der Öffnungen, vorzugsweise die in der unteren Haltelasche, den Dorn mit Spiel aufnimmt.

Zum weiteren Toleranzausgleich besitzt das Anschlussstück zwei rechtwinklig abgebogene Auflageschultern, welche auf einem Anschlag lagerbar sind.

Eine weitere Vereinfachung der Fertigung wird dadurch erreicht, dass der Dorn und der Anschlag Bestandteil eines gabelförmigen Aufnahmeelements sind. Ein solches Element kann am Boden des Spülgutträgers gehaltert sein. Außerdem ist es besonders vorteilhaft, wenn die Halterung des Aufnahmeelements bistabil ausgebildet ist, wobei in einer ersten stabilen Lage der Dorn zum Spülgutträgeräußeren von der Seitenwand beabstandet ist und in einer zweiten stabilen Lage der Dorn das Anschlussstück an die Seitenwand heranzieht. Hierdurch wird die Befestigung des Anschlussstücks am Spülgutträger vereinfacht. In der ersten stabilen Lage können die Auflageschultern auf dem Anschlag aufliegen.

Es ist zweckmäßig, wenn das Anschlussstück eine Führungsnut zur Aufnahme eines Anzugbolzens aufweist. Zur weiteren Vereinfachung des Kupplungsvorgangs ist es außerdem vorteilhaft, wenn die Führungsnut mit mindestens einer Erweiterung zur Aufnahme einer Positionierhilfe ausgestattet ist. Nachdem die Führungsnut den Anzugbolzen aufgenommen hat, muss zur Herstellung der Kupplung eine definierte Position zwischen Bolzen und Anschlussstück hergestellt werden. Dies wird dadurch erleichtert, dass das Anschlussstück auf der zum Träger gerichteten Seite ein Federelement zur Fixierung des Anzugbolzens besitzt.

Ausführungsbeispiele der Erfindung sind in den Zeichnungen rein schematisch dargestellt und werden nachfolgend näher beschrieben. Es zeigen
- Figur 1: einen Spülgutträger und ein dazugehöriges Anschlussstück in perspektivischer Darstellung;
- Figur 2: den Spülgutträger mit auf den Dorn aufgestecktem Anschlussstück und dem Aufnahmeelement in einer ersten stabilen Lage;
- Figur 3: den Spülgutträger mit auf den Dorn aufgestecktem Anschlussstück und dem Aufnahmeelement in einer zweiten stabilen Lage;
- Figuren 4 bis 6: das Anschlussstück in verschiedenen vergrößerten Ansichten;
- Figur 7: das Anschlussstück mit dem Anzugbolzen zu Beginn des Andockvorgangs;
- Figur 8: das Anschlussstück mit dem Anzugbolzen bei eingeschobenem Spülgutträger;
- Figuren 9 und 10: einen Spülgutträger mit einer weiteren Variante eines Aufnahmeelements in der ersten und zweiten stabilen Lage.

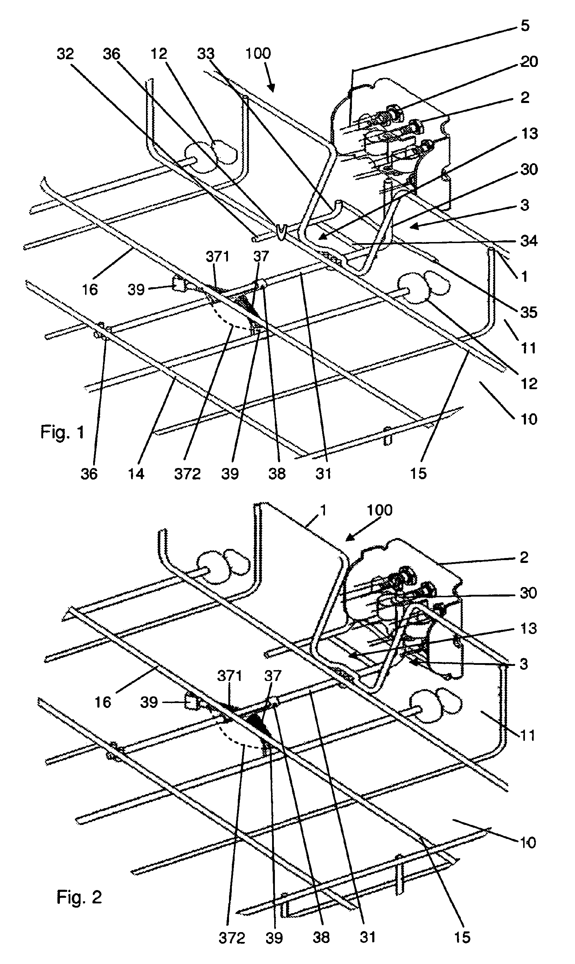

Der in den Figuren 1 bis 3 dargestellte Spülgutträger 100 dient zur Aufnahme von schlauchförmigem Reinigungsgut 5, insbesondere von Endoskopen. Hierzu wird er in den Spülraum eines Reinigungs- und Desinfektionsautomaten eingeschoben. Nach dem Einschieben erfolgt die Kopplung der Endoskope mit dem Spülflüssigkeits-, Desinfektionsflüssigkeits- und Trocknungsluftkreislauf des Automaten über eine Kupplungsvorrichtung.

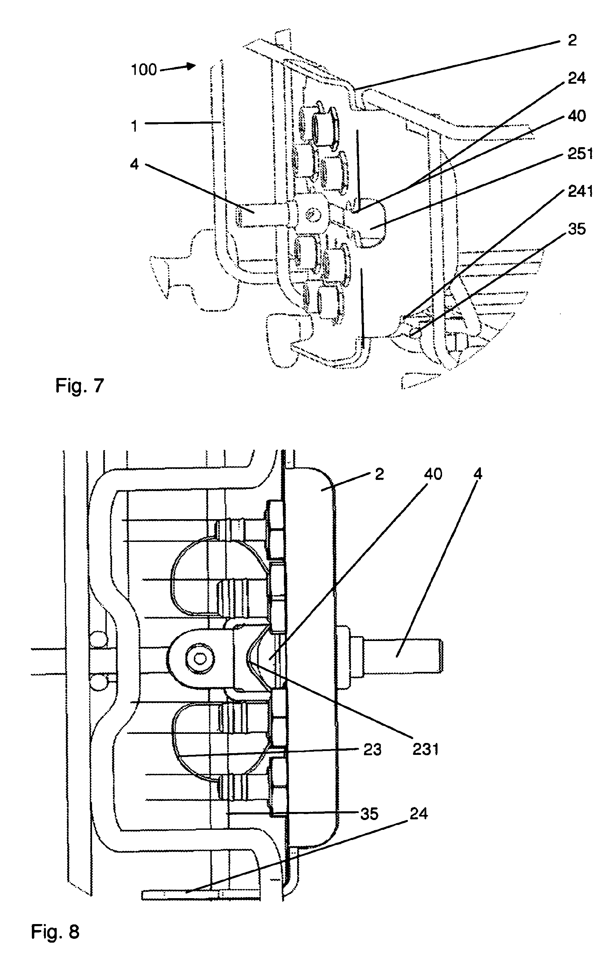

Ein möglicher Aufbau eines Reinigungs- und Desinfektionsautomaten und insbesondere der Aufbau einer Kupplungsvorrichtung ist beispielsweise aus der DE 10 2007 003 894 B4 bekannt und deswegen hier nicht näher dargestellt oder beschrieben. Die Figuren 7 und 8 zeigen lediglich den Anzugbolzen 4 der Kupplungseinrichtung, auf den weiter hinten eingegangen wird.

Der dargestellte Spülgutträger umfasst einen Spülkorb 1, dessen Boden 10 und umlaufende Seitenwände 11 eine gitterartige Struktur besitzen. Im Bodenbereich sind Gleitstücke 12 angeordnet, welche eine Bewegung des Korbs 1 auf nicht dargestellten Schienen innerhalb des Spülraums ermöglichen. Einer der Seitenwände 11, im gezeigten Ausführungsbeispiel die rechte, ist mit einer Aussparung 13 versehen. Diese dient zur Aufnahme eines Anschlussstücks 2, mit dessen Hilfe die Endoskope mit der Kupplungsvorrichtung verbunden werden. Die Endoskope sind in den Figuren nicht vollständig dargestellt, es sind lediglich einige Schlauchenden 5 angedeutet, die an Anschlussstutzen 20 befestigt werden. Um die Verbindung der Endoskope mit den Anschlussstutzen 20 zu erleichtern, kann das Anschlussstück 2 vom Spülkorb 1 gelöst werden, siehe Figur 1. Nach der Bestückung mit den Endoskopen wird das Anschlussstück 2 auf einen Dorn 30 aufgesteckt, der Bestandteil eines gabelförmigen Aufnahmeelements ist, im Folgenden als Gabel 3 bezeichnet. Die Gabel 3 umfasst einen langen Führungsdraht 31 mit dem rechtwinklig abgebogenen Dorn 30, einen kurzen Führungsdraht 32, welcher einen ebenfalls rechtwinkligen kurzen Schenkel 33 besitzt, eine Querstrebe 34, die die beiden waagerechten Schenkel der Führungsdrähte 31 und 32 verbindet, und einen Anschlagdraht 35, der am kurzen Schenkel 33 beginnend sich über den Dorn 30 hinaus erstreckt. Die Gabel ist in drei Ösen 36 geführt, welche an Streben 14 und 15 des Korbbodens 10 befestigt sind. Des Weiteren ist eine Schraubenfeder 37 durch eine Halteöse 38 an dem langen Führungsdraht 31 gesteckt und an ihren beiden Enden mittels drehbar gelagerter Halterungen 39 an einer Korbstrebe 16 fixiert. Der Abstand der beiden Halterungen 39 ist so ausgelegt, dass die Feder 37 unter Druck steht. Sie ist deshalb bestrebt, eine der beiden gestrichelt angedeuteten gekrümmten Positionen 371 oder 372 einzunehmen. Somit erzeugt die Feder 37 für den langen Führungsdraht 31 und hierüber für die gesamte Gabel 3 eine bistabile Lagerung.

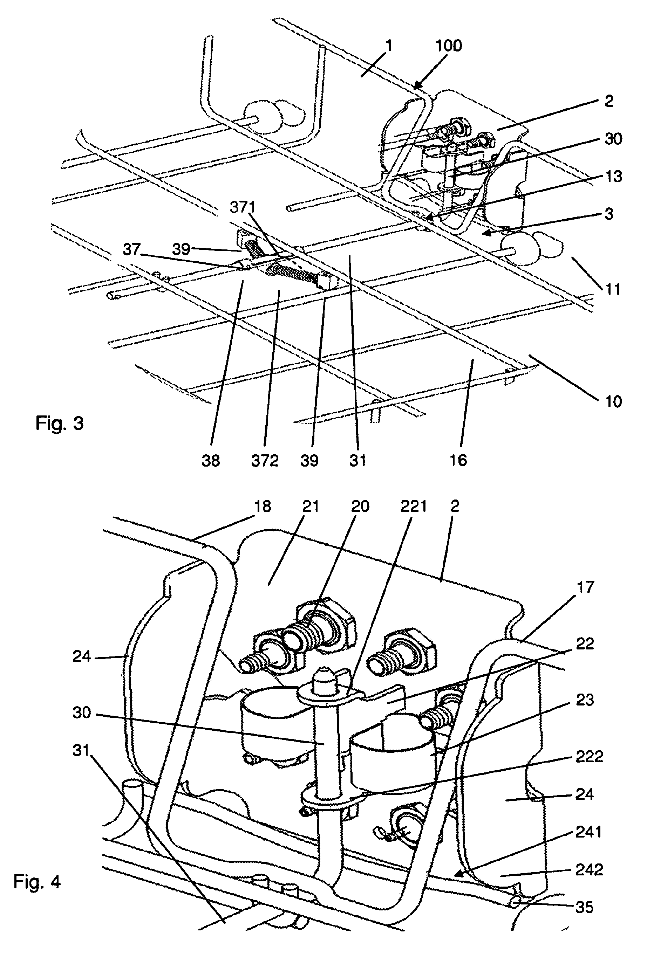

Die Figuren 1 und 2 zeigen die erste 371 der beiden stabilen Lagen 371 und 372, bei der der Dorn 30 aus der Aussparung 13 herausgefahren und zum Spülgutträgeräußeren von der Seitenwand 11 beabstandet ist. Diese Position erleichtert die Befestigung des Anschlussstücks 2 am Dorn 30. Anschließend drückt der Bediener das Anschlussstück 2 nach innen in die in der in Figur 3 dargestellten zweiten stabilen Lage 372. Dabei zieht der Dorn 30 das Anschlussstück 2 in die Aussparung 13 und an die betreffende Seitenwand 11 heran. In dieser Position ist das Anschlussstück 2 bereit für den später beschriebenen Andockvorgang.

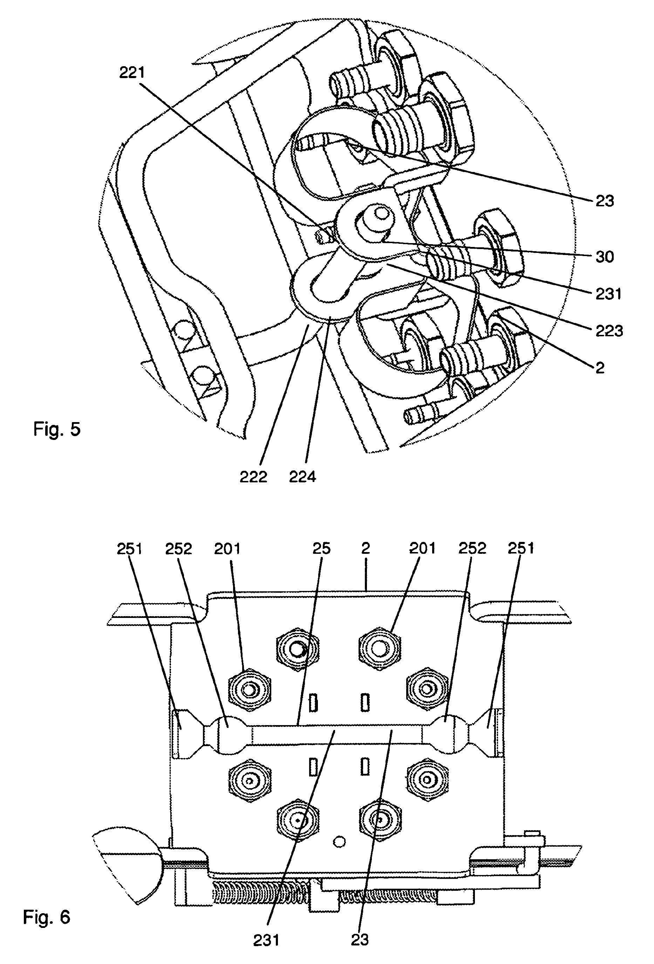

Die vergrößerten Darstellungen in den Figuren 4 und 5 zeigen das Anschlussstück 2 in dieser Position. Es ist erkennbar, dass das Anschlussstück 2 aus einer Andockplatte 21, einem Verstärkungsblech 22 mit einem Federelement 23 und den Anschlussstutzen 20 besteht. Die Andockplatte 21 ist an den Seiten rechtwinklig zu Auflageschultern 24 abgebogen. Es ist erkennbar, dass die Auflageschultern 24 mit ihren unteren Rundungen 241 so auf dem Anschlagdraht 35 aufliegen, dass sich ein geringes Spiel zwischen dem Draht 35 und den Eckbereichen 242 hinter den Rundungen 241 einstellt. Im oberen Bereich befinden sich die Auflageschultern 24 unter den waagerechten Streben 17 und 18, die den oberen Rand des Korbs 1 bilden. Damit bilden auch diese Streben 17 und 18 einen Anschlag, der ein Lösen des Anschlussstücks 2 vom Dorn 30 in dieser zweiten stabilen Lage verhindert.

Das Verstärkungsblech 22 besitzt zwei Haltelaschen 221 und 222, welche mit Öffnungen 223 und 224 versehen sind. Durch diese Öffnungen 223 und 224 wird der Dorn 30 gesteckt. In der Figur 5 ist erkennbar, dass die untere Öffnung 224 gegenüber dem Dorn 30 ein Übermaß von ca. 1 mm besitzt, die obere Öffnung 223 nimmt den Dorn 30 dagegen nur mit geringem Spiel auf. Alternativ können die beiden Öffnungen 223 und 224 aus zwei kreuzweise ausgerichteten Langlöchern bestehen. Es ist erkennbar, dass das Anschlussstück 2 in der zweiten stabilen Lage Spiel in alle Richtungen hat. Es kann sich einerseits um den Dorn 30 drehen und dabei je nach Drehrichtung mit einer Auflageschulter 24 auf dem Anschlagdraht 35 bis in den Eckbereich 242 gleiten. Andererseits ist aufgrund des Spiels zwischen Dorn 30 und Öffnung 224 auch eine Drehung um eine waagerechte Achse möglich. Das Anschlussstück 2 wird komplettiert durch ein in das Verstärkungsblech 22 eingelegtes Federelement 23 und durch die bereits vorbeschriebenen Anschlussstutzen 20.

Figur 6 zeigt die Rückseite des Anschlussstücks. Dort sind die Bohrungen 201 für die Anschlussstutzen 20 erkennbar. Außerdem ist eine Führungsnut 25 vorhanden, welche an ihren Enden v-förmig geöffnet ist. Hinter den Einführöffnungen 251 sind kreisförmige Erweiterungen 252 angeordnet. Das Federelement 23 ist hinter der Führungsnut angeordnet und besitzt eine von der Nut 25 weggerichtete Vertiefung 231, die auch in Figur 5 erkennbar ist.

Die Figuren 7 und 8 zeigen den Andockvorgang, durch den beim Einschieben des Spülkorbs 1 in den Spülraum das Anschlussstück 2 auf den Anzugbolzen 4 der Kupplungsvorrichtung geschoben wird. Dabei gerät der Kopf 40 des Bolzens 4 zunächst in die Erweiterung 251 der Führungsnut 25. Die spielbehaftete Lagerung des Anschlussstücks 2 stellt sicher, dass der Bolzen 4 immer die Führungsnut 25 findet. Durch die Lage des Bolzens 4 im Bezug zum Anschlussstück 2, dessen Höhenposition in dem Reinigungs- und Desinfektionsautomaten von der Korbgeometrie und von der Lage der Schienen bestimmt ist, wird erreicht, dass der Bolzen 4 beim weiteren Weg in der Nut 25 das Anschlussstück 2 anhebt. Dadurch werden das Spiel und damit die Beweglichkeit des Anschlussstücks 2 weiter vergrößert, was für den späteren, hier nicht beschriebenen Kupplungsvorgang wichtig ist. Der Andockvorgang ist beendet, wenn der Bolzen 4 in der Vertiefung 231 des Federelements 23 ruht. Dies wird dem Bediener durch eine Verringerung des Kraftaufwands signalisiert, der zum Einschieben des Korbs 1 notwendig ist. Anschließend kann der Kupplungsvorgang durchgeführt werden.

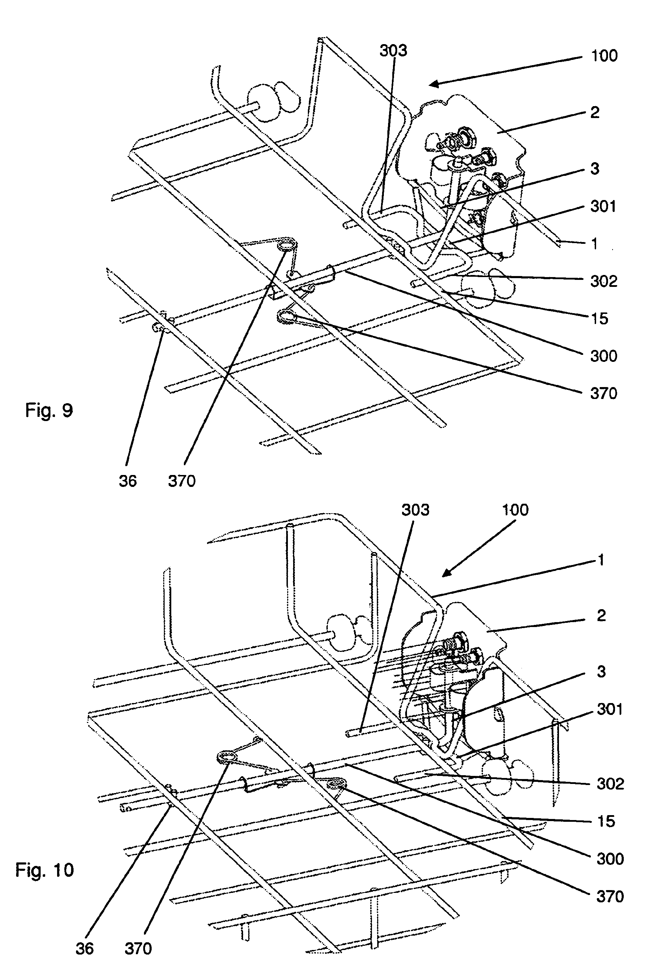

Die Figuren 9 und 10 zeigen eine alternative Ausführungsform eines gabelförmigen Aufnahmeelements 300 in der ersten stabilen Lage (Figur 9) und in der zweiten stabilen Lage (Figur 10). Hier wird die Schraubenfeder 37 durch zwei Schenkelfedern 370 ersetzt. Außerdem erfolgt die Führung der Gabel 300 am Korb durch zwei Schenkel 302 und 303 eines u-förmigen Führungsdrahts 301, die nicht in Ösen 36 gelagert sind, sondern frei an der Korbstrebe 15 gleiten.

## Patentansprüche

1. Spülgutträger (100) mit einem Anschlussstück (2) mit mehreren Anschlussstutzen (20) zur Aufnahme der Enden von mehreren schlauchförmigen Reinigungsgütern (5) und zur Verbindung mit einer Kupplungsvorrichtung einer den Spülgutträger (100) aufnehmenden Spülmaschine, wobei der Spülgutträger (100) einen Boden (10) und mindestens eine Seitenwand (11) besitzt, und wobei das Anschlussstück (2) während des Spülbetriebs an der Seitenwand (11) angeordnet ist,
**dadurch gekennzeichnet,**
**dass** das Anschlussstück (2) lösbar an der Seitenwand (11) befestigt ist.

2. Spülgutträger (100) nach Anspruch 1,
**dadurch gekennzeichnet,**
**dass** das Anschlussstück (2) in der Betriebslage des Spülgutträgers (100) in vertikaler Richtung in eine Aussparung (13) der Seitenwand (11) einsetzbar ist.

3. Spülgutträger (100) nach einem der Ansprüche 1 oder 2,
**dadurch gekennzeichnet,**
**dass** das Anschlussstück (2) aus Blech hergestellt ist.

4. Spülgutträger (100) nach Anspruch 2 und 3,
**dadurch gekennzeichnet,**
**dass** das Anschlussstück (2) mindestens eine Haltelasche (221, 222) mit einer Öffnung (223, 224) besitzt, welche von einem in der Aussparung (13) angeordneten, sich in der Betriebslage des Spülgutträgers (100) in vertikaler Richtung erstreckenden Dorn (30) aufnehmbar ist.

5. Spülgutträger (100) nach Anspruch 4,
**gekennzeichnet durch** eine obere Haltelasche (221) und eine untere Haltelasche (222), wobei mindestens eine der Öffnungen (223, 224), vorzugsweise die Öffnung (224) in der unteren Haltelasche (222), den Dorn (30) mit Spiel aufnimmt.

6. Spülgutträger (100) nach mindestens einem der Ansprüche 3 bis 5,
**dadurch gekennzeichnet,**
**dass** das Anschlussstück (2) zwei rechtwinklig abgebogene Auflageschultern (24) besitzt, welche auf einem Anschlag (35) lagerbar sind.

7. Spülgutträger (100) nachn Anspruch 4 und 6,
**dadurch gekennzeichnet,**
**dass** der Dorn (30) und der Anschlag (35) Bestandteil eines gabelförmigen Aufnahmeelements (3) sind.

8. Spülgutträger (100) nach Anspruch 7,
**dadurch gekennzeichnet,**
**dass** das Aufnahmeelement (3) am Boden (10) des Spülgutträgers (100) gehalten wird.

9. Spülgutträger (100) nach Anspruch 8,
**dadurch gekennzeichnet,**
**dass** die Halterung des Aufnahmeelements (3) bistabil ausgebildet ist, wobei in einer ersten stabilen Lage der Dorn (30) zum Spülgutträgeräußeren von der Seitenwand (11) beabstandet ist und in einer zweiten stabilen Lage der Dorn (30) das Anschlussstück (2) an die Seitenwand (11) heranzieht.

10. Spülgutträger (100) nach mindestens einem der Ansprüche 6 bis 9,
**dadurch gekennzeichnet,**
**dass** in der ersten stabilen Lage die Auflageschultern (24) auf dem Anschlag (35) aufliegen.

11. Spülgutträger (100) nach mindestens einem der Ansprüche 1 bis 10,
**dadurch gekennzeichnet,**
**dass** das Anschlussstück (2) eine Führungsnut (25) zur Aufnahme eines Anzugbolzens (4) der Kupplungsvorrichtung aufweist.

12. Spülgutträger (100) nach Anspruch 11,
**dadurch gekennzeichnet,**
**dass** die Führungsnut (25) mit mindestens einer Erweiterung (252) zur Aufnahme einer Positionierhilfe ausgestattet ist.

13. Spülgutträger (100) nach einem der Ansprüche 11 oder 12,
**dadurch gekennzeichnet,**
**dass** das Anschlussstück (2) auf der zum Spülgutträger (100) gerichteten Seite ein Federelement (23) zur Fixierung des Anzugbolzens (4) besitzt.

## Claims

1. Washware carrier (100) comprising a connecting piece (2) which comprises a plurality of connecting branches (20) for receiving the ends of a plurality of tube-shaped items to be cleaned (5) and for connecting to a coupling device of a rinsing machine which accommodates the washware carrier (100), the washware carrier (100) having a base (10) and at least one side wall (11) and the connecting piece (2) being arranged on the side wall (11) during the washing operation, **characterised in that** the connecting piece (2) is detachably fastened to the side wall (11).

2. Washware carrier (100) according to claim 1, **characterised in that** the connecting piece (2) can be inserted into a recess (13) in the side wall (11) in a vertical direction in the operating position of the washware carrier (100).

3. Washware carrier (100) according to either claim 1 or claim 2, **characterised in that** the connecting piece (2) is made from sheet metal.

4. Washware carrier (100) according to claims 2 and 3, **characterised in that** the connecting piece (2) has at least one retaining tab (221, 222) comprising an opening (223, 224), which can be received by a mandrel (30) which is arranged in the recess (13) and extends in a vertical direction in the operating position of the washware carrier (100).

5. Washware carrier (100) according to claim 4, **characterised by** an upper retaining tab (221) and a lower retaining tab (222), at least one of the openings (223, 224), preferably the opening (224) in the lower retaining tab (222), receiving the mandrel (30) with play.

6. Washware carrier (100) according to at least one of claims 3 to 5, **characterised in that** the connecting piece (2) has two supporting shoulders (24) which are bent at right angles and can be mounted on a stop (35).

7. Washware carrier (100) according to claims 4 and 6, **characterised in that** the mandrel (30) and the stop (35) are components of a forked receiving element (3).

8. Washware carrier (100) according to claim 7, **characterised in that** the receiving element (3) is held on the base (10) of the washware carrier (100).

9. Washware carrier (100) according to claim 8, **characterised in that** the holder of the receiving element (3) is formed in a bistable manner, the mandrel (30) towards the outside of the washware carrier being spaced apart from the side wall (11) in a first stable position, and the mandrel (30) pulling the connecting piece (2) onto the side wall (11) in a second stable position.

10. Washware carrier (100) according to at least one of claims 6 to 9, **characterised in that** the support shoulders (24) rest on the stop (35) in the first stable position.

11. Washware carrier (100) according to at least one of claims 1 to 10, **characterised in that** the connecting piece (2) comprises a guide groove (25) for receiving a pulling bolt (4) of the coupling device.

12. Washware carrier (100) according to claim 11, **characterised in that** the guide groove (25) is equipped with at least one expansion (252) for receiving a positioner.

13. Washware carrier (100) according to either claim 11 or claim 12, **characterised in that** the connecting piece (2), on the side directed towards the washware carrier (100), has a spring element (23) for fixing the pulling bolt (4).

## Revendications

1. Porte-vaisselle (100) avec une pièce de raccordement (2) avec plusieurs tubulures de raccordement (20) pour loger les extrémités de plusieurs produits à nettoyer (5) tubulaires et pour le raccordement à un dispositif d'accouplement d'une machine à laver logeant le porte-vaisselle (100), le porte-vaisselle (100) possédant un fond (10) et au moins une paroi latérale (11), et la pièce de raccordement (2) étant disposée sur la paroi latérale (11) pendant le mode lavage, **caractérisé en ce que** la pièce de raccordement (2) est fixée de façon détachable sur la paroi latérale (11).

2. Porte-vaisselle (100) selon la revendication 1,
**caractérisé en ce que** la pièce de raccordement (2), dans la position de fonctionnement du porte-vaisselle (100), peut être introduite en direction verticale dans un évidement (13) de la paroi latérale (11).

3. Porte-vaisselle (100) selon une des revendications 1 ou 2,
**caractérisé en ce que** la pièce de raccordement (2) est réalisée en tôle.

4. Porte-vaisselle (100) selon les revendications 2 et 3,
**caractérisé en ce que** la pièce de raccordement (2) possède au moins une attache de retenue (221, 222) avec une ouverture (223, 224) qui peut être reçue par une broche (30) qui s'étend en direction verticale dans la position de fonctionnement du porte-vaisselle (100) et qui est disposée dans l'évidement (13).

5. Porte-vaisselle (100) selon la revendication 4,
**caractérisé par** une attache de retenue supérieure (221) et une attache de retenue inférieure (222), au moins une des ouvertures (223, 224), de préférence l'ouverture (224) dans l'attache de retenue inférieure (222), recevant la broche (30) avec du jeu.

6. Porte-vaisselle (100) selon au moins une des revendications 3 à 5,
**caractérisé en ce que** la pièce de raccordement (2) possède deux épaulements d'appui (24) recourbés à angle droit qui peuvent être supportés sur une butée (35).

7. Porte-vaisselle (100) selon les revendications 4 et 6,
**caractérisé en ce que** la broche (30) et la butée (35) sont des composants d'un élément de réception (3) en forme de fourche.

8. Porte-vaisselle (100) selon la revendication 7,
**caractérisé en ce que** l'élément de réception (3) est retenu sur le fond (10) du porte-vaisselle (100).

9. Porte-vaisselle (100) selon la revendication 8,
**caractérisé en ce que** la retenue de l'élément de réception (3) est réalisée de façon bistable, la broche (30) vers l'extérieur du porte-vaisselle (100) étant, dans une première position stable, éloignée de la paroi latérale (11) et, dans une deuxième position stable, la broche (30) attirant la pièce de raccordement (2) sur la paroi latérale (11).

10. Porte-vaisselle (100) selon au moins une des revendications 6 à 9,
**caractérisé en ce que**, dans la première position stable, les épaulements d'appui (24) reposent sur la butée (35).

11. Porte-vaisselle (100) selon au moins une des revendications 1 à 10,
**caractérisé en ce que** la pièce de raccordement (2) présente une rainure de guidage (25) pour recevoir un axe de traction (4) du dispositif d'accouplement.

12. Porte-vaisselle (100) selon la revendication 11,
**caractérisé en ce que** la rainure de guidage (25) est équipée d'au moins un élargissement (252) pour la réception d'une aide au positionnement.

13. Porte-vaisselle (100) selon une des revendications 11 ou 12,
**caractérisé en ce que** la pièce de raccordement (2) possède, sur le côté dirigé vers le porte-vaisselle (100), un élément à ressort (23) pour la fixation de l'axe de traction (4).
